# EUROPEAN PATENT APPLICATION

(11) **EP 3 479 857 A1**
(43) Date of publication of application: **08.05.2019**
(21) Application number: 17199879.2
(22) Date of filing: 03.11.2017
(51) Int. Cl.: A61M 5/28, A61M 5/315

(54) **SEAL FOR LOCKING A SYRINGE**

(71) Applicant: Fagron B.V., 3062 ME Rotterdam (NL)
(72) Inventor: DIJKERS, Eli Carl Ferre, 3021 JP Rotterdam (NL); STOB, Paul Herman, 9713 TN Groningen (NL); VEENENDAAL, Reinier Floris Hendricus, 3061 DZ Rotterdam (NL); SEGHER, Natasja, 9140 Temse (BE); JAGER, Esther Helina, 6811 CH Arnhem (NL); VERHOEVEN, Louise Maria, 3541 RK Utrecht (NL)
(74) Representative: Nederlandsch Octrooibureau

(57) **Abstract**

The current invention in most general terms relates to ready-to-use prefilled syringes. More in particular, the invention relates to a seal for locking the plunger of a syringe at a pre-selected position relative to the syringe barrel. The invention also relates to an assembly comprising the seal of the invention and a syringe. According to the invention, the syringe is typically a prefilled syringe provided with a capped needle in the assembly of the invention. Furthermore, the invention relates to a syringe provided with a seal of the invention. The invention also relates to a mold for molding the seal of the invention. In addition, the invention relates to a computer readable medium comprising computer executable instructions and 3D data representing the seal of the invention, which instructions and 3D data, when loaded on a computer system connected to a 3D printer, allows to 3D print the seal of the invention.

## Description

### FIELD OF THE INVENTION

The current invention in most general terms relates to ready-to-use prefilled syringes. More in particular, the invention relates to a seal for locking the plunger of a syringe at any pre-selected position relative to the syringe barrel, by clamping the plunger shaft and the grip of the syringe barrel together. The invention also relates to an assembly comprising the seal of the invention and a syringe. According to the invention, the syringe is typically a pre-filled syringe provided with a capped needle in the assembly of the invention. According to the invention, the seal of the invention is typically for use in sealing a syringe prefilled with a medicament. Furthermore, the invention relates to a syringe provided with a seal of the invention. The invention also relates to a mold for molding the seal of the invention. In addition, the invention relates to a computer readable medium comprising computer executable instructions and 3D data representing the seal of the invention, which instructions and 3D data, when loaded on a computer system connected to a 3D printer, allows to 3D print the seal of the invention.

### BACKGROUND OF THE INVENTION

Fluids such as liquid medicaments can be relatively expensive. For example, the procedure of manufacturing a medicament can be costly, and/or the active pharmaceutical ingredient can be costly. In addition, use of *e.g.* medicaments generally requires a high standard of care, in-depth knowledge and well-trained skills. That is to say, not only the act of administration of a medicament such as in particular an injectable medicament, requires extreme care and a high level of skills. In addition and not to a lesser level of importance, also the administered amount and dose of a medicament is of extreme importance to the well-being and health of the subject to whom the medicament is administered and is of extreme importance in view of the (desired) effect of the medicament and perhaps also in view of the prevention or control of adverse side effects coming with the use of the medicament in a body, *e.g.* a human body. Both administering by injection a too small amount of a medicament to a subject, and to the opposite a too large amount, may induce serious consequences in the injected subject. Great effort is therefore required in order to prevent errors with regard to the administered amount and dose of a medicament to a subject.

In addition to the aforementioned requirements and circumstances that all should be optimized for a proper use of a medicament in a subject, administered by injection, also the quality and effectivity and volume in *e.g.* a prefilled syringe, of a medicament released by a manufacturer for use in a subject, should be stable and stored for a sufficiently amount of time. Moreover, such a prefilled syringe should be stabilized and made resistant with regard to any mechanical forces exposed to the syringe during *e.g.* transport and storage. Quality, effectivity and volume of a medicament are all compromised in time by external influences such as temperature, drops and rises in temperature, exposure to ultraviolet light, *etc., etc.* Most importantly, in a prefilled syringe, such as a prefilled syringe provided with a needle, also the material of the syringe as well as of the needle may exert an influence on the medicament in the syringe. Leakage from the syringe may occur. The medicament may become less efficacious during time due to for example chemical reactions and/or physical interactions between the medicament and syringe/needle material. For example, liquid medicament entering the needle after filling, during storage and as a result of movement of the plunger due to any mechanical forces acting upon the prefilled syringe during *e.g.* transport, may result in one or more of a number of devastating outcomes with regard to the quality and effectivity of *e.g.* a liquid medicament such as an aqueous pharmaceutical composition.

Furthermore, an additional problem encountered with providing a ready-to-administer pharmaceutical composition in a syringe with a needle is keeping the syringe and thus the pharmaceutical composition free from contamination with any microbes during storage and transport up to the moment at which the pharmaceutical composition is to be administered to a subject. Moreover, providing a prefilled syringe filled with a pharmaceutical composition with a needle comes with the risk for spoiling and/or contaminating the pharmaceutical composition when connecting the needle to the syringe and during subsequent storage and transport of the ready-to-use syringe. However, also storing and transporting prefilled syringes without a needle, such that a needle has to be connected to the prefilled syringe shortly before administration of the pharmaceutical composition to a subject, comes with the similar risk for spoiling the pharmaceutical composition and/or contaminating the pharmaceutical composition with any microbe when connecting the needle to the syringe.

One approach to address one or several of the above outlined safety issues, *etc.,* is to store a fluid in a syringe, *e.g.* a liquid medicament in a prefilled syringe, by locking the predetermined volume of the medicament in the syringe by application of a seal for fixation of the syringe plunger to the barrel of the syringe. This way, the volume of medicament is largely prevented from leaking out or being pushed out of the syringe, *e.g.* through the needle end of the syringe, since the plunger is immobilized. In addition, prefilling a syringe with a predetermined unit dose of a medicament and then locking the syringe by use of a seal greatly contributes to the avoidance of errors with regard to administering the correct dose to a subject. Last but not least, conserving a fluid such as a liquid medicament in a syringe by use of a seal provides the possibility to check and control whether the syringe remained untampered during, *e.g.* production, transport, storage, up to the act of breaking the seal and administering the *e.g.* liquid medicament to a patient.

However, improving on the quality of a fluid by applying a current seal on a syringe filled with the fluid comes with drawbacks. For example, currently available devices such as seals for syringes cannot be easily removed from the syringe without substantial effort required from the user, or such devices appear not to be tamper proof, and moreover, such devices can only be positioned at a certain predetermined and fixed position at the plunger relative to the syringe barrel strongly hampering flexibility of the device with regard to the volume of pharmaceutical composition to be selected for prefilling a syringe. Removing a seal requires substantial force and/or is only possible when using two hands. Furthermore, breaking a seal results in the seal falling apart in several parts, *e.g.* parts with sharp edges, which introduces the risk for injuries by cutting or for tearing *e.g.* the gloves of the user in *e.g.* a surgeons operation room, *etc.* In addition, such parts of the seal after breaking the seal introduces for example the risk for contacting the (opened) body of a patient in an operation room with (contaminated) particles.

Seals for syringes are known in the art. Dutch patent NL1038569 describes a seal for clamping the shaft of a plunger of a syringe at a predetermined fixed position. The seal of NL1038569 is designed for use with syringes having a plunger shaft with a selected single shape and size and having a grip at the barrel of the syringe, which grip has a selected single shape and size, which selected shape and size of the plunger shaft and of the grip fit with notches and apertures of the seal. Different syringes with different plunger shafts and/or different grips require different seals, according to NL1038569. The seal of NL1038569 is either provided as a seal which can be reversibly locked once clamped to a syringe, or is provided as a seal which is irreversibly locked once clamped to a syringe. Apparently, breaking the seal results in the seal falling apart in at least two parts, based on the drawings of NL1038569.

European patent application EP04735043 relates to means for limiting the dose that may be dispensed by a syringe, and describes a seal for mounting on the plunger of a syringe, at a position at any location along the plunger shaft. The seal consists of two hinging parts both comprising notched portions for receiving the arms of the cross of a plunger-shaft formed as a cruciform, when the seal is in locked position. The notched portions are provided with clamping pins for embedding into the plunger to prevent sliding of the seal relative to the plunger. The two hinging parts each comprise the complementary part of a lock. The seal of EP04735043, once applied to a plunger of a syringe, permanently prevents retraction of the plunger head relative to the syringe barrel beyond a predetermined distance.

United Kingdom patent GB2377176 relates to a seal for use in controlling the administration of a drug by a syringe, wherein the seal is locatable upon the syringe. The seal is not tamper proof, or tamper evident, and the seal is selectively movable between a locked position in which the syringe is prevented from moving the plunger of the syringe, and an unlocked position which allows the plunger of the syringe being moved. According to GB2377176, the seal is releasably secured to the syringe such that the seal is in locked position, and the seal is configured to unlock from the plunger such that the plunger can be moved. The seal comprises two pivotally connected parts, said parts comprising a releasable lock means. The two hinging parts of the seal both comprise notched portions for receiving the arms of the cross of a plunger-shaft formed as a cruciform, when the seal is in locked position as well as when the seal is unlocked such that the plunger of the syringe is moveable.

International patent application WO 01/023017 relates to a seal for locking the plunger of a syringe at a predetermined position relative to the barrel of the syringe, thereby preventing movement of the syringe plunger relative to said barrel. The seal both encapsulates the grip of the syringe plunger and receives the grip of the syringe barrel, when the seal is applied to the syringe plunger, thereby limiting the dynamic range of locked positions of the plunger relative to the barrel. The seal of WO 01/023017 is not tamper evident, or tamper proof. According to WO 01/023017, different seals are to be manufactured, the seals having different sizes to accommodate various syringe sizes and volumes of medicament to be contained by the syringes.

However, the seals of the prior art do not provide for:
i) a convenient method of placing such seal to a (prefilled) syringe, wherein positioning and fixation of the seal at a selected location of the plunger, *i.e.* clamping the plunger and the barrel of the syringe together, does not introduce a risk for developing injuries to the nerves and muscles and tendons of persons working with the seals during prolonged periods of clamping multiple seals to syringes, such as for example repetitive strain injury;
ii) breaking the seal without burdensome effort and without the risk for damaging the skin of the hand or gloves;
iii) breaking the seal without the risk for contacting and contaminating a patient's body in the operation room with particles coming loose of the seal during breaking the seal;
iv) confirming beyond a certain level of doubt that the seal is untampered, *i.e.* that the seal is tamper proof, or tamper evident; and
v) use with a multitude of varying syringes with regard to the shape and the volume *c.q.* size of said syringes.

Furthermore, current devices such as currently available seals are applicable for use with a single type and shape of syringe and syringe plunger, thereby limiting the broad applicability of such seals to a large extent.

Therefore, an improved seal that fills in the shortcomings of current seals is highly desired in the field of *e.g.* manufacturing of precious solutions such as costly liquid medicaments, such solutions subsequently brought in a barrel such as a syringe barrel.

### SUMMARY OF THE INVENTION

The current invention relates to a seal that provides a solution to many, if not all, of the shortcomings of seals currently applied in the field of manufacturing syringes prefilled with any precious fluid such as a liquid medicament, for example an aqueous pharmaceutical composition. The seal of the invention is a tampered proof seal. The seal is placed and positioned on the plunger shaft of a syringe and on the grip of the syringe barrel with great ease, without the requirement of substantial skills and force. Herewith, the use of the seal of the invention provides for a convenient method of placing a seal to a (prefilled) syringe. Clamping such a seal of the invention to a syringe does not introduce a risk for developing injuries to for example muscles and tendons and nerves of the hand and arm of the persons providing syringes with the seal of the invention, even when seals are clamped to syringes for prolonged periods of time during a day, and for prolonged periods of time during weeks and months. Clamping a seal of the invention to a syringe only requires the closure of a single lock, said lock for example comprising a bulged pin - slot configuration. Related to that, the seal of the invention is easily removable from the syringe, even with one hand, without applying a high amount of force. Removing the seal of the invention results in the seal remaining as a single part, without sharp edges, *etc.,* thereby reducing the risk for cutting skin or gloves of the user, and thereby reducing the uncontrolled scattering of seal pieces in *e.g.* the area near to a subject such as a subject in an operation room. Furthermore, the seal of the invention is applicable with a wide array of different syringes having plungers with shafts having varying shapes and sizes and a multitude of syringe barrel volumes and variously sized and shaped barrel grips. As a result, greater flexibility is afforded to the user of the seal of the invention by the use of the seal.

The current invention relates to a seal for locking a plunger of a syringe, the seal comprising
- a first part and a second part;
   a hinge arranged to hinge the first and second parts,
   each part provided with
      -- at least one aperture for receiving a grip of the syringe,
      -- a notched portion for receiving the plunger,
- the seal further comprising
   -- a lock arranged at the first part and second part opposite to the hinge;
      wherein said lock is provided with
         --- a pin releasably connected to the first part through a removable seal, said pin provided with a bulged end portion in an end portion; and
         --- a slot provided in the second part, arranged
            to engage the bulged end portion of the pin in a locked position.

By application of a seal of the invention with a prefilled syringe, movement of the first and second parts of the seal from the locked position to an open position of the seal is prevented.

In an embodiment, the seal according to the invention has a lock with a slot with an opening which opening is provided with a rim portion, preferably a resilient rim portion.

In an embodiment, the seal according to the invention comprises a lock with a slot, wherein the rim portion of opening of the slot is provided with at least one notch, preferably two notches located at opposite sides of the rim, more preferably four notches positioned at a mutual angle of about 90° relative to one another.

In an embodiment, the seal according to the invention comprises a lock with a slot, wherein the rim portion of opening of the slot is provided with at least one notch, preferably two notches located at opposite sides of the rim, more preferably four notches located at an angle of about 90° relative to one another.

In one embodiment, the seal according to the invention comprises a lock comprising a pin, wherein the bulged end portion in the end portion of the pin is an enlarged head encompassing the end portion, or is a rim, or is a harpoon of a harpoon closure encompassing the end portion, or is the first half of a snap closure for engagement with the second half of a snap closure located in the slot, or is the first half of an interlocking closure for engagement with a second half of an interlocking closure located in the slot.

In one embodiment, the seal according to the invention comprises a lock with a slot, wherein the pin of the slot is provided with a resilient bulged portion.

In one embodiment, the seal according to the invention comprises a lock with a slot, wherein the pin of said slot is provided with a handle arranged to release the pin from the first part of the seal.

In one embodiment, the seal according to the invention comprises at least one of a thermosetting polymer material selected from the group comprising polystyrene and polycarbonate, and/or one of a thermoplastic polymer material selected from acrylonitrile butadiene styrene and acrylonitrile styrene acrylate, preferably, the seal is manufactured from polystyrene. Of course, the seal of the invention may also comprise other polymers or a polymer blend, according to the invention.

In one embodiment, The seal according to the invention is manufactured from a single type of thermosetting polymer material selected from polystyrene and polycarbonate, or is manufactured from a single type of thermoplastic polymer material selected from acrylonitrile butadiene styrene and acrylonitrile styrene acrylate, preferably, the seal is wholly manufactured from polystyrene. In one embodiment, the seal of the invention is manufactured from a single polymer blend known in the art.

In one embodiment, the seal according to the invention comprises a first part and a second part, each part provided with a notched portion for receiving the plunger of a syringe, wherein the notched portion of said first and second part is configured for step less receiving a plunger shaft of the plunger.

In one embodiment, the seal according to the invention comprises a first part and a second part, wherein said first and second part each is provided with at least two apertures for receiving the grip of the syringe, which grip is part of the barrel of the syringe.

An aspect of the invention is a seal for locking a plunger of a syringe, the seal comprising
- a first and second part;
   a hinge arranged to hinge said first and second parts,
   each part provided with
      -- at least one aperture for receiving a grip of the syringe,
      -- a notched portion for receiving the plunger.

In a preferred embodiment, the seal according to the invention further comprises
- a lock arranged at the first and second part of the seal opposite to the hinge arranged to hinge said first and second parts;
   wherein said lock is provided with
      -- a pin releasably connected to the first hinged part via a removable seal, said pin
         provided with a bulged end portion of the pin, and
      -- a slot provided in the second hinged part, to engage the bulged end portion of the pin.

In a preferred embodiment, the seal according to the invention further comprises
- a lock arranged at the first and second part of the seal opposite to the hinge arranged to hinge said first and second parts;
   wherein said lock is provided with
   -- a single pin releasably connected to the first hinged part via a removable seal, said pin
      provided with a bulged end portion of the pin, and
   -- a single slot provided in the second hinged part, to engage the bulged end portion of the pin.

In one embodiment, the seal according to the invention comprises a first and second part, wherein said first and second part each is provided with at least two apertures for receiving the grip of a syringe and wherein the apertures are arranged for receiving grips with different dimensions and/or with a different shape.

In one embodiment, the seal according to invention comprises a first part and a second part, each part provided with a notched portion for receiving the plunger of a syringe, wherein the notched portions of the first and second parts form a circular opening when the seal is in the locked position, wherein the notched portions are arranged conform a circular shape for receiving the plunger-shaft of the plunger of the syringe.

In one embodiment, the seal according to the invention comprises a first part and a second part, each part provided with a notched portion for receiving the plunger of a syringe, wherein the notched portions of the first and second parts form a circular opening when the seal is in the locked position, wherein the notched portions are arranged conform a circular shape for receiving the plunger-shaft of the plunger of the syringe, and wherein each notched portion further comprises at least two clamping pins positioned at a mutual angle of 90° such that the at least four clamping pins are respectively positioned at a mutual angle of 90° in the circular opening formed by notched portions when the seal is in the locked position. Such clamping pins are provided for embedding into the shaft of the syringe plunger to aid in prevention of sliding of the seal relative to the plunger shaft, once the seal is clamped to a syringe.

In one embodiment, the seal according to the invention comprises a first part and a second part, each part provided with a notched portion for receiving the plunger of a syringe, wherein the notched portions of the first and second part are each provided with a notch and a first and second recess portion in the first part and a first and second recess portion in the second part, such that each of the notches and opposite recess portions of the notched portions are arranged conform a cruciform for receiving a plunger-shaft, when the seal is in locked position.

In one embodiment, the seal according to the invention comprises a first part and a second part, each part provided with a notched portion for receiving the plunger of a syringe, wherein the notched portions of the first and second parts form a circular opening when seal is in the locked position, wherein the notched portions are arranged to receive conform a circular shape for receiving the plunger-shaft of the plunger, and wherein the notched portions of the first and second part are each provided with a notch and a first and second recess portion in the first part and a first and second recess portion in the second part, such that each of the notches and opposite recess portions of the notched portions are arranged conform a cruciform for receiving a plunger-shaft, when the seal is in locked position.

A third aspect of the current invention is an assembly comprising the seal according to the invention and a syringe.

Preferably, in the assembly of the invention, the syringe is further provided with a needle connected to a nozzle of the syringe, said needle preferably provided with a releasable cap.

A fourth aspect of the current invention is a syringe provided with a seal according to the invention.

A fifth aspect of the current invention is a mold for molding the two parts of the seal of the invention. Preferably, the seal is made of a single material such as a polymer or a polymer blend, preferably the seal is made of polystyrene.

A sixth aspect of the current invention is a computer readable medium comprising computer executable instructions and 3D data representing the seal of the invention, which, when loaded on a computer system connected to a 3D printer, allows to 3D print the two parts of the seal.

A further aspect of the current invention is a computer system comprising a processor, a memory and a 3D printer, which processor is arranged to execute instructions stored in said memory, for 3D printing of the two parts of the seal of the invention by said 3D printer.

Other features and advantages of the present invention will become apparent from the following description of the preferred embodiments, which, when taken in conjunction with the accompanying drawings, illustrate the principles of the current invention. The description of the preferred embodiments should not be interpreted as limiting the present invention in any way.

### BRIEF DESCRIPTION OF THE DRAWINGS

Figure 1. Perspective back view of a seal according to a first embodiment of the invention, in open position, showing two hinged parts of the seal and elements of a lock connected to each part.
Figure 2. Perspective front view of a seal according to a first embodiment of the invention, as in Figure 1, in locked position. A multitude of slits in the casing of the seal is visible, for receiving the grip of syringe barrels having a variety of sizes and shapes.
Figure 3. Perspective back view of a first part of the seal according to a first embodiment of the invention, showing the pin part of the lock of the seal.
Figure 4. Perspective back view of a second part of the seal according to a first embodiment of the invention, showing the part of the lock of the seal for receiving the pin of the lock.
Figure 5. Perspective back view of a seal according to a first embodiment of the invention, as in Figure 1, in locked position.
Figure 6. Front view of a seal according to a first embodiment of the invention, as in Figure 2, in locked position.
Figure 7. Bottom view of a seal according to the invention.
Figure 8. Front view of a seal according to an alternative embodiment of the invention, in closed and locked position, the seal comprising notches for receiving a syringe plunger having a substantially circular cross section.
Figure 9. Front view of a seal according to yet another alternative embodiment of the invention, in closed and locked position, the seal comprising notches for receiving a syringe plunger having a cross section with the shape of a cruciform.
Figure 10. Front view of a seal according to a first embodiment of the invention, in closed and locked position, the seal comprising notches for receiving a syringe plunger having a cross section with the shape of a cruciform and/or having a substantially circular cross section.
Figure 11. Perspective view of an assembly according to the invention comprising a seal according to a first embodiment of the invention in closed and locked position and a syringe comprising a barrel and a plunger. The cross section of the plunger shaft is substantially a cruciform.
Figure 12. Perspective view of an alternative assembly according to the invention comprising a seal according to a first embodiment of the invention in closed and locked position and a syringe comprising a barrel and a plunger. The cross section of the plunger shaft has the shape of a cruciform.
Figure 13. Perspective view of yet another alternative assembly according to the invention comprising a seal according to a first embodiment of the invention in closed and locked position and a syringe comprising a barrel and a plunger. The plunger shaft is composed of two perpendicularly oriented planes with ridges in the corners of the perpendicularly positioned planes.

### DETAILED DESCRIPTION OF THE PREFERRED EMBODIMENTS

The term "tamper proof", also referred to as "tamper resistance", has its normal scientific meaning throughout the application and here refers to the resistance to tampering (intentional malfunction or sabotage) by a user of *e.g.* a product which is meant to remain untampered, *e.g.* up till the intended specific use of said product. Typically, a product is made tamper proof by applying a seal to the product. Then, a broken or torn seal is a measure or indication for the initially sealed product not being untampered anymore.

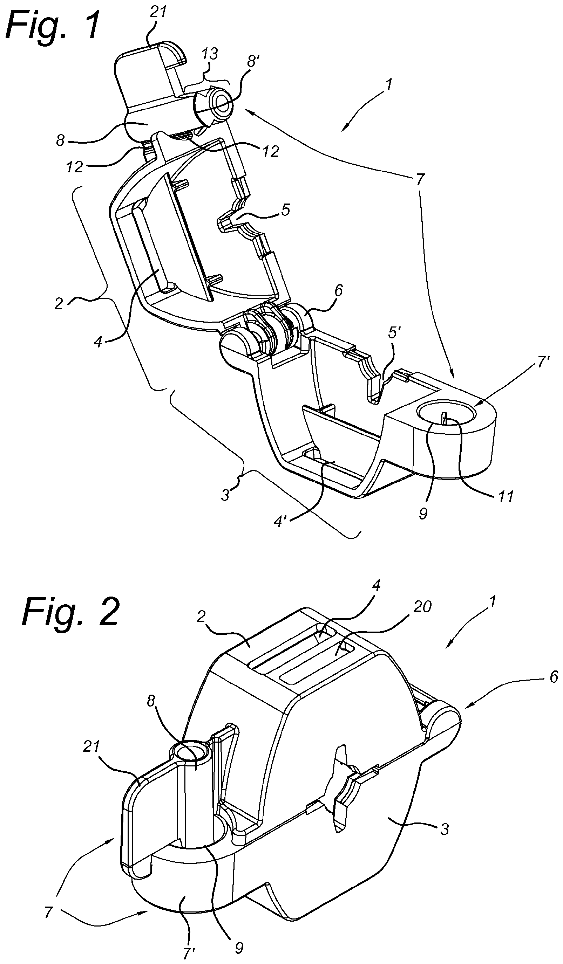

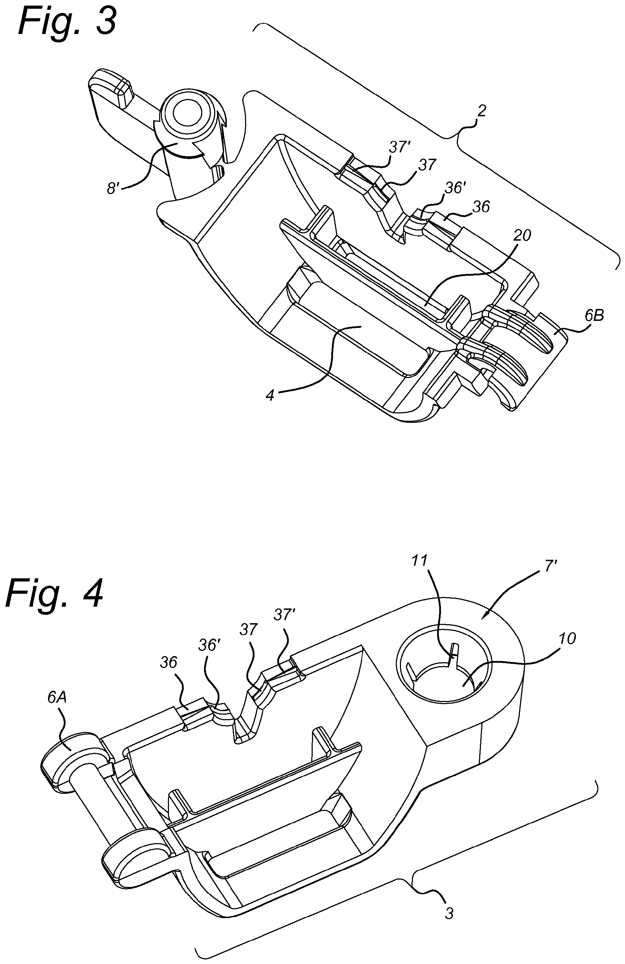

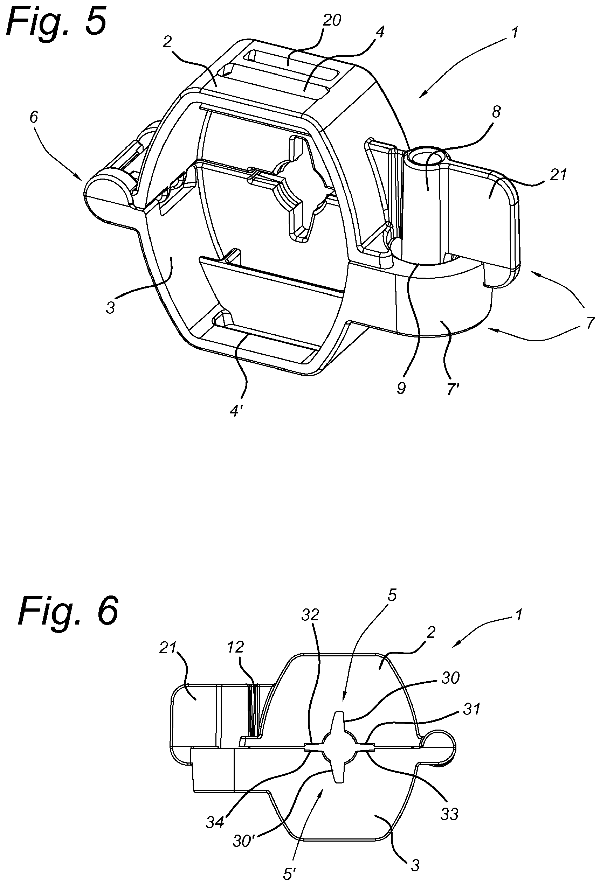

In accordance with a first embodiment of the invention and referring to Figures 1 to 10, there is illustrated and shown a seal 1, 50, 60 according to the invention. The seal 1, 50, 60 is for locking a plunger 14 of a syringe 15, 15' by clamping the shaft of the plunger 17, 25, 26 to the grip 16 of the syringe said grip located at the syringe barrel 18. The seal 1, 50, 60 comprising
- a first part 2, 47 and a second part 3, 46;
   a hinge 6, 6a, 6b arranged to hinge the first and second parts 2, 3, 47, 46,
   each part provided with
      -- at least one aperture 4, 4', 20 for receiving a grip 16 of the syringe,
      -- a notched portion 5, 5' for receiving the plunger 14,
- the seal 1, 50, 60 further comprising
   -- a lock 7 arranged at the first part and second part 2, 3, 47, 46 opposite to the hinge 6;
      wherein said lock 7 is provided with
         --- a pin 8 releasably connected to the first part 2, 47 through a removable seal 12, said pin 8 provided with a bulged end portion 8' in an end portion 13; and
         --- a slot 7' provided in the second part 3, 46, arranged
   to engage the bulged end portion 8' of the pin 8 in a locked position.

The lock 7 is arranged such that movement of the first and second parts 2, 3, 47, 46 from the locked position to an open position of the seal is prevented once the seal is locked. That is to say, in the locked position of the seal movement of the two first and second parts 2, 3, 47, 46 from the locked position to the open position of the seal is prevented when an opening 9 of the slot 7' is engaged by the bulged portion 8' of the pin 8.

In one embodiment, the seal 1, 50, 60 of the invention is provided such that the first part and the second part 2, 3, 47, 46 and the hinge 6, 6a, 6b are integrated into a single piece according to the invention.

The seal 1, 50, 60 is provided with a removable seal 12, which is for example a breakable seal or a tearable seal, such that the seal 1, 50, 60 is intentionally removable from a syringe to which the seal is clamped, by breaking or tearing the removable seal 12. According to one embodiment, the removable seal 12 is tearable or breakable without leaving sharp edges and/or sharp parts after tearing or breaking the seal. It is an important aspect of the current invention that tearing or breaking the removable seal 12 results in the seal 1, 50, 60 being removable from a syringe, while said seal remains a single part, without the seal falling apart in more than one pieces or elements. Since the seal 1, 50, 60 remains as one single part with the parts 2, 3 remaining attached to each other via hinge 6, the seal of the invention provides for various benefits when compared to seals currently applied to *e.g.* syringes. That is to say, removal of the seal of the invention from a syringe comes with a reduced risk for (uncontrolled) scattering of (tiny, sharp, harmful) pieces of seal, to the benefit for *e.g.* a user of the sealed syringe or a patient in an operation room or a hospital setting. The user of the sealed syringe is exposed to less risk for torn gloves such as surgeon gloves when tearing the seal, and exposed to less risk for cutting the skin of *e.g.* the fingers and the hand. Since tearing the seal of the invention does not provide multiple pieces of seal that could uncontrollable fall at any surface such as the (opened) body of a patient in an operation room, the seal of the invention introduces less risk for contacting the patient's body with parts of the seal and thus lowers the risk for contaminating the patient's body with any microbe that may be adhered to parts of the seal, when compared to current seals that fall apart uncontrollable when opened.

In one embodiment, the removable seal 12 is tearable leaving edges after tearing the seal which edges have a surface that introduces a smaller risk for tearing *e.g.* surgeon gloves when contacting or moving such gloves along the surface of said edges, when compared to the edges that are exposed when tearing seals currently available.

Thus, the pin 8 remains engaged in the slot 7' upon tearing/breaking the removable seal 12 thereby disconnecting the pin 8 from the first portion 2, 47 of the seal 1, 50, 60. In other words, the pin 8 remains engaged in the slot 7' when the removable seal 12 is removed such that the pin 8 is disconnected from the first portion 2, 47 of the seal 1, 50, 60.

One way of establishing this permanent engagement of pin 8 in slot 7', is by providing the opening 9 of slot 7' with a rim portion 10, which rim portion is preferably a resilient rim portion 10. By application of this rim portion 10 in the opening 9 of the slot 7', engagement of the pin 8 with the bulged end portion 8' by the opening 9 results in an irreversible locking of the seal 1, 50, 60. Alternatively, or additively, in an embodiment of the invention, the rim portion 10 of the opening 9 of the slot 7' is provided with at least one notch 11, preferably two notches 11 located at opposite sides of the rim, more preferably four notches 11 located at a mutual angle of about 90° relative to one another. By application of these one or more notches, either or not in combination with a resilient rim portion 10, engagement of the bulged end portion 8' of the pin 8 is facilitated, while at the same time release of the bulged end portion 8'out of the opening 9 is prevented. Of course, the bulged end portion 8' may have one or more of many possible shapes and sizes, that all are fit to the purpose of being able to engage the opening 9 while being not able to be relieved from the slot 7'once the bulged end portion 8' passed the opening 9.

Thus, according to the invention, in a series of equivalent embodiments the seal 1, 50, 60 of the invention comprises a pin 8 releasably connected to the first part 2, 47 of the seal, wherein the bulged end portion 8' in the end portion 13 of the pin 8 is either an enlarged head encompassing the end portion 13, or is a rim (not shown), or is a harpoon of a harpoon closure (not shown) encompassing the end portion 13, or is the first half of a snap closure for engagement with the second half of a snap closure located in the slot 7' (not shown), or is the first half of an interlocking closure for engagement with a second half of an interlocking closure located in the slot 7' (not shown), to name a few possible means for irreversibly locking pin 8 in slot 7'. According to the invention, the applicable means and method for closing and locking the seal 1, 50, 60 of the invention are thus numerous, as long as the seal is tamper proof with regard to said closing and locking means. The closing and locking means of the seal of the invention are resistant to tampering; the seal of the invention is removed from a syringe by breaking/tearing the removable seal 12, according to the invention. Preferred is a seal 1, 50, 60 of the invention comprising closing and locking means consisting of a pin 8 releasably connected to the first part 2, 47 of the seal, wherein the pin 8 comprises a bulged end portion 8' configured for engagement with the opening 9 of slot 7' (See for example Figure 1, 3, 4 and 7).

The seal 1, 50, 60 can be provided with a pin 8 which pin is provided with a handle 21 arranged to release the pin 8 from the first part 2 of the seal 1, 50, 60 by facilitating the breaking/tearing of the removable seal 12. Typically, upon application of the handle 21, the user of the seal 1, 50, 60 is able to tear/break the removable seal 12 upon applying a reduced amount of force, due to the leverage, when compared to the force required to remove current seals applied to syringes or when compared to the force required to break or tear the removable seal 12 when the pin 8 is not provided with such a handle 21 or the like. Thus, in one embodiment, the pin 8 is provided with a handle 21, wherein the pin 8 is released from the first part 2 of the seal by rotating the pin 8 along its longitudinal axis over for example between about 45° to 180°, preferably less, or preferably over for example between 90° and 120° such as about 100°, such that the removable seal 12 is torn or broken from the first part 2 and such that the pin 8 is disconnected from the first part 2 while remaining engaged in the slot 7'.

In a preferred embodiment, the part 2 of the seal 1, 50, 60 is provided with a pin 8 comprising a resilient bulged portion 8'. Such a resilient bulged portion 8' is for example made of a rubber or a polymer such as a polymer blend or a single polymer such as polystyrene. Applying a resilient bulged portion 8' provides the benefit of a more easy engagement of the pin 8 by the slot 7', requiring less force by the user of the seal.

It will be appreciated that the seal 1, 50, 60, and in particular its casing, *i.e.* the parts 2 and 3 of the seal, can be manufactured from many different materials. However, manufacturing the seal of the invention from a polymer or polymer blend is preferred. Thus, the seal 1, 50, 60 of the invention comprises for example at least one of a thermosetting polymer material selected from the group comprising polystyrene and polycarbonate, and/or comprises for example one of a thermoplastic polymer material selected from acrylonitrile butadiene styrene and acrylonitrile styrene acrylate. Of course, also a seal produced from combinations of said thermosetting polymers and/or thermoplastic polymers is part of the invention. For the ease of production and for the ease of tearing and/or breaking the removable seal 12, preferably, the seal 1, 50, 60 is manufactured from a single type of polymer or polymer blend, most preferably at least part of the seal, including the removable seal 12 is made of polystyrene.

As said, although many (polymer) materials are applicable for producing a seal of the invention, in particular embodiments, the seal 1, 50, 60 of the invention is manufactured from a single type of thermosetting polymer material selected from polystyrene and polycarbonate, or is manufactured from a single type of thermoplastic polymer material selected from acrylonitrile butadiene styrene and acrylonitrile styrene acrylate. In a particularly preferred embodiment, the seal 1, 50, 60 is wholly manufactured from polystyrene. The inventors noticed that the first and second part of the seal of the invention are conveniently produced from polystyrene, and that tearing or breaking the removable seal 12 is then relatively easy, requiring minimum force. Most importantly, breaking or tearing of the removable seal 12 made of polystyrene resulted in smooth and blunt surfaces at the pin 8 and at the part 2 of the seal once the removable seal 12 was torn or broken. Such smooth and blunt surfaces prevented cutting of the skin of the fingers of the operator who torn/broke the removable seal, and prevented tearing of gloves worn in *e.g.* the operation room.

Referring to the embodiments illustrated in Figures 1 to 6 and 11 to 13, the first and second parts 2, 3, 47, 46 of the seal 1, 50, 60 of the invention are provided with a notched portion 5, 5', which notched portions are configured such that the shaft 17, 25, 26 of a syringe plunger is steplessly receiveable by the seal. Receiving the plunger shaft steplessly provides the benefit of freedom of providing a prefilled syringe with seemingly endless variation of the volume of *e.g.* the liquid medicament such as a pharmaceutical composition, *e.g.* for use in the treatment of a cancer, diabetes, myocardial infarction, sepsis, *etc.* The seal of the invention for a prefilled syringe is for example particularly suitable for providing a pharmaceutical composition for use in the treatment of degenerative eye disease such as a pharmaceutical composition for use in the eye of a patient suffering from macular degeneration. That is to say, first a syringe can be filled with any predetermined desired volume of a fluid, *e.g.* a liquid medicament, and then the seal 1, 50, 60 can be clamped to the plunger shaft and to the grip of the syringe, without restrictions with regard to the position of the plunger shaft relative to the syringe barrel. Herewith, a seal 1, 50, 60 is provided for universal application with regard to the volume of liquid medicament present in the syringe barrel, since the seal is configured for receiving a plunger shaft pulled out of the syringe barrel to any possible extent. Thus, for example, in certain embodiments, the syringe is first filled with a volume of a liquid medicament of between about 25 microliter and 2 ml or higher, such as 25 microliter, 40, 50, 60, 70, 80, 90, 100, 150, 200, 500 microliter, 1, 1,5 ml, or with any volume of a liquid medicament in between these indicated volumes. Of course even smaller volumes and also larger volumes can be contained by syringes of applicable volume, such that a seal of the invention is subsequently attached to the syringe plunger and grip of the syringe. For example, in an embodiment, a syringe suitable for containing at maximum 2 ml is prefilled with 1 ml of a liquid medicament, and subsequently the seal 1, 50, 60 of the invention is clamped to the plunger shaft of the syringe and concomitantly to the grip of the syringe. In alternative embodiments, the seal 1, 50, 60 is applied to syringes having a capacity for containing at maximum for example 500 microliter, 1 ml, 5 ml, 10 ml, 20 ml, 25 ml, 50 ml or even 100 ml of a fluid such as an aqueous pharmaceutical composition. In one embodiment, the seal of the invention is clamped to a prefilled syringe, said prefilled syringe being provided with a needle.

Many, if not all syringes used in the art for providing a liquid medicament, are provided with a syringe barrel comprising a grip at the barrel-end configured for receiving the syringe plunger. Typically, such a grip of a syringe comprises a transverse flange portion. Thus, in a preferred embodiment, the at least one aperture 4, 4', 20 in the first and second parts 2, 3, 47, 46 of the seal for receiving a grip 16 of a syringe, is an aperture configured for receiving a transverse flange portion of a grip of the barrel 18 of a syringe. It is appreciated by the skilled person that the aperture(s) can have different sizes and shapes when configured for receiving grips of syringes having variant sizes and/or shapes.

In a particularly preferred embodiment of the invention, the first and second part 2, 3, 47, 46 of seal 1, 50, 60 each is provided with at least two apertures 4, 4', 20 for receiving the grip 16 of the syringe 15, 15'. Preferably, the first and second part 2, 3, 47, 46 of seal 1, 50, 60 each is provided with two apertures 4, 4', 20, wherein each aperture is configured to receive a grip with a certain size and/or shape, and in combination the apertures are configured to receive various grips with varying sizes and/or shapes. Such a seal 1, 50, 60 of the invention comprising at least two differently sized and/or shaped apertures for receiving a variety of differently sized and/or shaped grips of syringes, provides for a highly flexible application of the seal, with virtually no restriction as to the type and size and shape of syringe to which the seal is clamped. This way, the seal 1, 50, 60 of the invention is applicable with many if not all syringes currently applied in healthcare, *etc.,* for administering for example liquid medicament to a subject, *e.g.* a human subject. Thus, the seal 1, 50, 60 according to the invention comprises a first and a second part 2, 3, 47, 46 each provided with at least two apertures 4, 4', 20 for receiving a grip 16 of a syringe 15, 15' and wherein the separate apertures in each part are arranged for receiving grips with different dimensions and/or with a different shape.

Referring now to Figure 1-7, 11-13, there is shown a seal 1, 50, 60 according to an embodiment of the invention. A seal 1, 50, 60 is provided for locking a plunger 14 of a syringe 15, 15', the seal 1, 50, 60 comprising
- a first and second part 2, 3, 47, 46;
   a hinge 6 arranged to hinge said first and second parts,
   each part provided with
      -- at least one aperture 4, 4', 20 for receiving a grip 16 of the syringe,
      -- a notched portion 5, 5' for receiving the plunger 14.

The notched portion 5, 5' is configured to receive the plunger shaft 17, 25, 26 of a plunger 14 of a syringe such that the seal 1, 50, 60 is clamped to said plunger shaft when the seal is in locked position.

In a preferred embodiment, the seal 1, 50, 60 of the invention comprises
- a lock 7 arranged at the first and second part 2, 3, 47, 46 opposite to the hinge 6;
   wherein said lock 7 is provided with
      -- a pin 8 releasably connected to the first hinged part 2, 47 via a removable seal 12, said pin 8
         provided with a bulged end portion 8' of the pin 8, and
      -- a slot 7' provided in the second hinged part 3, 46, to
   engage the bulged end portion 8' of the pin 8.

In a preferred embodiment, the seal 1, 50, 60 of the invention comprises
- a lock 7 arranged at the first and second part 2, 3, 47, 46 opposite to the hinge 6;
   wherein said lock 7 is provided with
      -- a single pin 8 releasably connected to the first hinged part 2, 47 via a removable seal 12, said pin 8
         provided with a bulged end portion 8' of the pin 8, and
      -- a single slot 7' provided in the second hinged part 3, 46, to engage the bulged end portion 8' of the pin 8.

Once the seal 1, 50, 60 of the invention is clamped to the shaft of a syringe plunger and concomitantly to the grip of the syringe barrel, such that the seal is in locked position, the plunger is immobilized relative to the syringe barrel. Thus, the current invention provides for a tamper proof seal 1, 50, 60 for locking the shaft 17 of a plunger 14 at any pre-selected position, such as for example a predetermined position 17' of the plunger shaft in the barrel 18 of a syringe 15, 15'. A volume of *e.g.* a liquid medicament contained in a prefilled syringe sealed with a seal 1, 50, 60 of the invention is stored and liquid cannot enter the needle that is optionally provided at the supply end of the syringe barrel. This way, the volume of a liquid in the prefilled syringe locked with a seal 1, 50, 60 of the invention, is stable and the quality of the liquid such as a liquid medicament is not influenced by for example exposure to needle material.

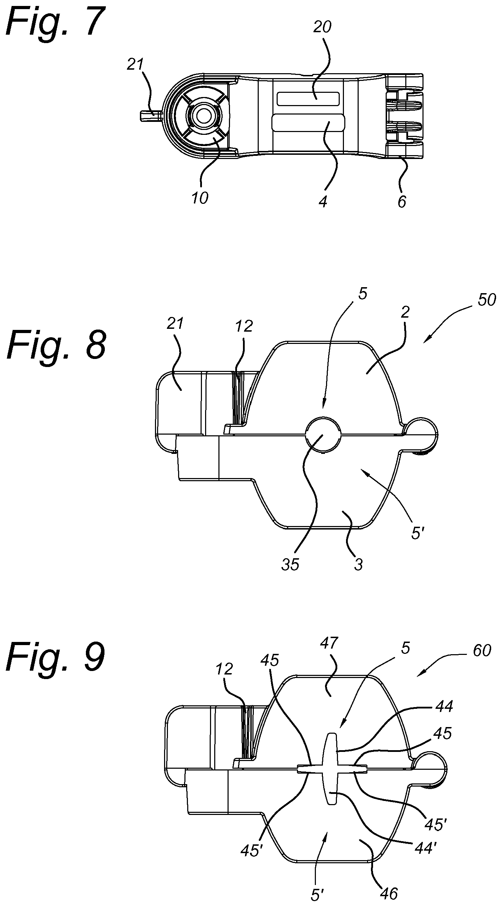

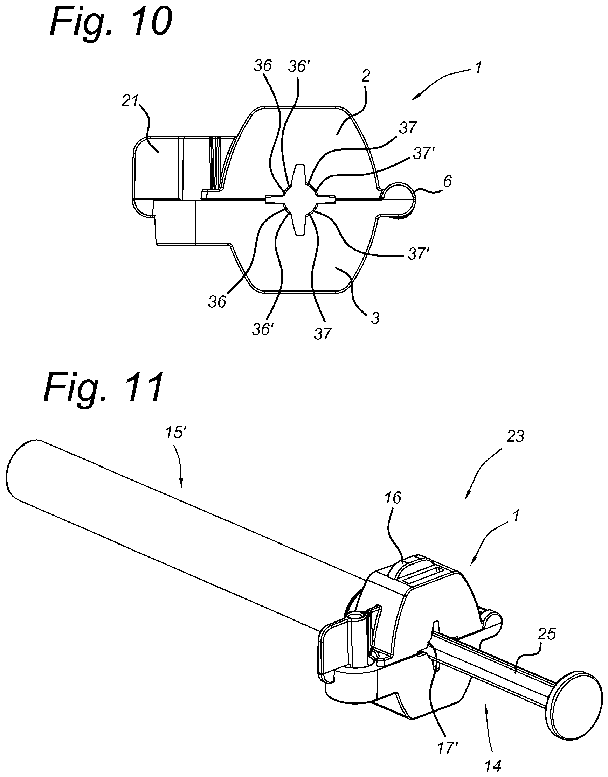

Now turning to a further embodiment, illustrated in Figures 8 and 11, there is shown a syringe 15' provided with a shaft 25 having a substantially cylindrical shape, preferably a cylindrical shape. In this embodiment, the seal 50 of the invention comprises a first part 2 and a second part 3, provided with notched portions 5, 5' forming a substantially circular opening, preferably a circular opening 35 when seal 50 is in the locked position, wherein the notched portions 5, 5' are arranged conform a circular shape for receiving the plunger-shaft 25 of the plunger 14 of the syringe 15'. It is evident for the skilled person that either a seal 50 is provided suitable for receiving a cylindrically shaped plunger-shaft having a certain fixed radius, *i.e.* a plunger shaft with an essentially circular cross-section, or that seal 50 is provided suitable for receiving various different cylindrically shaped plunger-shafts having a varying radius, *i.e.* various plunger shafts each with an essentially circular cross-section wherein the diameter of these cross-sections may vary between different shafts, wherein the seal fits with shafts having relatively smaller radius as well as with shafts having a larger radius, according to the invention. Of course, providing a seal arranged for receiving shafts of plungers with a certain range of shaft radius, is preferred since such a seal of the invention is more universally applicable.

In order to improve the grip of the seal on the shaft of a syringe plunger, when the seal is clamped to a substantially cylindrically shaped plunger-shaft and thus in locked position, in one embodiment, the seal 1, 50 of the invention comprises a first part 2 and a second part 3, each part provided with a notched portion 5, 5' for receiving the plunger 14 of a syringe 15, wherein the notched portions 5 5' of the first and second parts 2, 3 form a circular opening 35 when the seal 1, 50 is in the locked position, wherein the notched portions 5, 5'are arranged conform a circular shape for receiving the plunger-shaft 25 of the plunger 14 of the syringe 15, and wherein each notched portion 5, 5' further comprises at least two clamping pins 36, 36', 37, 37' positioned at a mutual angle of 90° such that the at least four clamping pins are respectively positioned at a mutual angle of 90° in the circular opening 35 formed by notched portions 5, 5' when the seal 1, 50 is in the locked position. In locked position of the seal 1, 50, the clamping pins aid in fixation and immobilization of the plunger at a (pre-selected) position relative to the syringe barrel, by improving the grip of the seal on the substantially cylindrical plunger shaft of the syringe. It is also part of the invention that the notched portions 5, 5' are provided with at least one, such as two, three, four, five, six, *etc.,* clamping pins, with the aim of further strengthen the grip of the seal in locked position on the plunger. Preferably, the clamping pins are an integral part of the part 2 and part 3 of the seal 1, 50, and preferably, said clamping pins, such as ridges, bulbs, or the like, are made of the same material as the whole of part 2 and part 3 of the seal. Typically, a seal of the invention is completely made of polystyrene, but also other polymers and polymer blends are suitable materials for manufacturing a seal of the invention comprising notched portions with clamping pins.

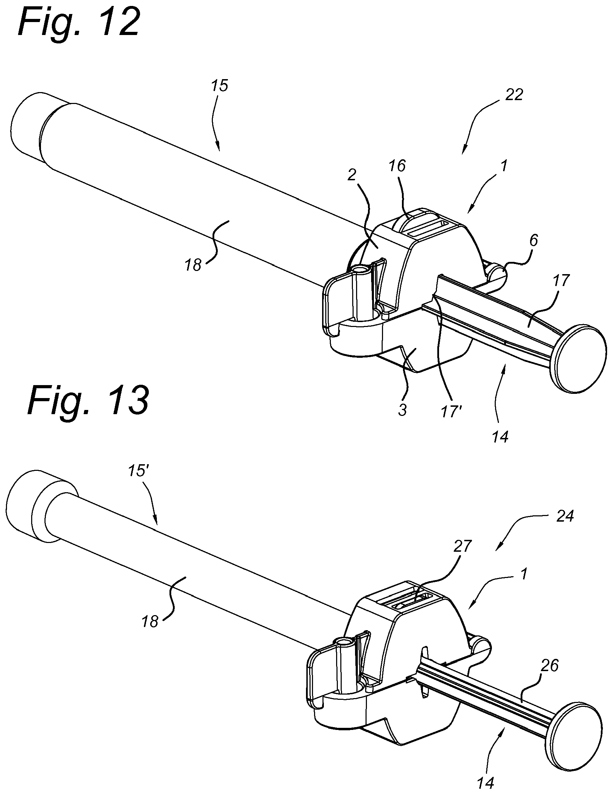

In an alternative embodiment, illustrated by Figure 9 and 12, the seal 60 of the invention comprises a first part 47 and a second part 46, provided with notched portions 5, 5' forming a recess arranged for receiving a cruciform shaped body, wherein the notched portions 5, 5' of the first and second part 47, 46 are each provided with a notch 44, 44' and a first and second recess portion 45 in the first part 47 and a first and second recess portion 45' in the second part 46, such that each of the notches 44, 44' and opposite recess portions 45, 45' of the notched portions are arranged conform a cruciform for receiving a plunger-shaft 17, 26, when the seal 60 is in locked position. Since a substantial number of syringes provided as prefilled syringes containing a liquid, such as a liquid medicament for use in *e.g.* healthcare, comprises a plunger shaft having the cross-sectional shape of a cruciform, the seal 60 of this embodiment is applicable for a wide array of currently used syringes. It is appreciated that the dimensions of the notched portions 5, 5' are adaptable to the individual size and/or shape of such syringes having a plunger shaft having the cross-sectional shape of a cruciform. Of course, it is preferred if a single seal 1, 60 of the invention is adapted for receiving various plunger shafts having the cross-sectional shape of a cruciform, wherein the shape and dimensions of such cruciform varies within certain ranges. This way, the seal 1, 60 of the current embodiment is applicable for receiving the plunger shaft of a wide range of different syringes with a variety of cross-sectional shaped plungers having different sizes and shapes.

In a particularly preferred embodiment, the seal 1 of the invention comprises a first part 2 and a second part 3 provided with notched portions 5, 5' forming a recess arranged for receiving a cruciform shaped body as well as arranged for receiving a cylindrically shaped body, when the seal is in locked and closed position. Reference is made to Figures 1, 2, 5, 6, 10, 11 and 13, with this regard. Thus, it is preferred that the seal 1 of the invention comprises a first part 2 and a second part 3, each part comprising a notched portion 5, 5', wherein the notched portions 5, 5' of the first and second parts 2, 3 form a circular opening 35 when seal 1 is in the locked position, wherein the notched portions 5, 5' are arranged conform a circular shape for receiving the plunger-shaft 25 of the plunger 14 of a syringe, and wherein the notched portions 5, 5' of the first and second part 2, 3 are each provided with a notch 30, 30' and a first and second recess portion 31, 32 in the first part 2 and a first and second recess portion 33, 34 in the second part 3, such that each of the notches 30, 30' and two opposite recess portions 31, 33 and two opposite recess portions 32, 34 of the notched portions are arranged conform a cruciform for receiving a plunger-shaft, when the seal 1 is in locked position. This way, the seal 1 is arranged for receiving virtually all plunger shafts nowadays in use for *e.g.* healthcare applications, said plunger shafts having a substantially cylindrical cross section or having a cross section as a cruciform, or having a plunger shaft with a cross section which is a combination of a cylindrical cross section and a cross section as a cruciform. It is one of the many advantages of the seal of the current invention, that the seal is applicable with virtually all syringes in use in the field of medicine, healthcare, *etc., etc.*

In a particularly preferred embodiment, the seal 1 of the invention arranged for receiving a plunger shaft having a cylindrical cross section, and arranged conform a cruciform for receiving a plunger shaft having a cross section as a cruciform, and arranged for receiving a plunger shaft with a cross section which is a combination of a cylindrical cross section and a cross section as a cruciform, further comprises a first and second part 2, 3 of seal 1, each part provided with at least two apertures 4, 4', 20 for receiving a grip 16 of a syringe 15, 15'. Preferably, the first and second part 2, 3 of seal 1 each is provided with two apertures 4, 4', 20, wherein each aperture is configured to receive a grip with a certain size and/or shape, and in combination the apertures are configured to receive various grips with varying sizes and/or shapes. Combining the flexibility of the seal 1 with regard to the size and shape of the plunger shaft that fits the notched portion 5, 5' when the seal is in locked position, with the flexibility of the seal 1 related to the size and shape of the at least two apertures 4, 4', 20 for receiving the grip 16 of the syringe barrel 18 of the syringe 15, 15', reveals a seal 1 which is applicable for sealing a wide range of different syringes with regard to the volume which can be contained by the syringe, the size and shape of the plunger of the syringe, as well as the size and shape of the syringe barrel and grip of the syringe. The inventors thus provided with the current seal 1 of the invention for a universal seal applicable with most of the currently applied syringes in *e.g.* the field of healthcare involving administering predetermined and fixed volumes of *e.g.* liquid medicaments to subjects in need thereof, *e.g.* human patients.

One aspect of the invention relates to a seal 1, 50, 60 for locking a plunger 14 of a syringe 15, 15', the seal comprising
- a first part and a second part 2, 3, 47, 46;
   a hinge 6, 6a, 6b arranged to hinge the first and second parts 2, 3, 47, 46,
   each part provided with
      -- at least one aperture 4, 4', 20 for receiving a grip 16 of the syringe,
      -- a notched portion 5, 5' for receiving the plunger 14,
- the seal 1, 50, 60 further comprising
   -- a lock 7 arranged at the first part and second part 2, 3, 47, 46 opposite to the hinge 6;
      wherein said lock 7 is provided with
         --- a pin 8 releasably connected to the first part 2, 47 through a removable seal 12, said pin 8 provided with a bulged end portion 8' in an end portion 13; and
         --- a slot 7' provided in the second part 3, 46, arranged
            to engage the bulged end portion 8' of the pin 8 in a locked position,
wherein the first and second part 2, 3, 47, 46 each is provided with at least two apertures 4, 4', 20 for receiving the grip 16 of a syringe 15, 15'.

In one embodiment, the seal 1, 50, 60 according to the invention comprises the first and second parts, wherein the first and second part 2, 3, 47, 46 each is provided with at least two apertures 4, 4', 20 for receiving the grip 16 of a syringe 15, 15', wherein the apertures are arranged for receiving grips with different dimensions and/or with a different shape.

In one embodiment, the seal 1, 50, 60 according to the invention comprises the first and second parts, wherein the notched portion 5, 5' of the first and second part 2, 3, 47, 46 is configured for stepless receiving a plunger shaft 17, 25, 26 of the plunger 14.

In one embodiment, the seal 1, 50, 60 according to the invention comprises the first and second parts 2, 3, 47, 46, wherein the first and second part each is provided with at least two apertures 4, 4', 20 for receiving the grip 16 of the syringe 15, 15'.

In one embodiment, the seal 50 according to the invention comprises the first and second parts, wherein the notched portions 5, 5' of said first and second parts 2, 3, 47, 46 form a circular opening 35 when the seal 50 is in the locked position, wherein the notched portions 5, 5' are arranged conform a circular shape for receiving the plunger-shaft 25 of the plunger 14 of the syringe 15'.

In one embodiment, the seal 50 according to the invention comprises the first and second parts, wherein each notched portion 5, 5' comprises at least two clamping pins 36, 36', 37, 37' positioned at a mutual angle of 90° such that the at least four clamping pins are respectively positioned at a mutual angle of 90° in the circular opening 35 formed by notched portions 5, 5' when the seal 50 is in the locked position.

In one embodiment, the seal 60 according to the invention comprises the first and second parts, wherein, wherein the notched portions 5, 5' of the first and second part 47, 46 are each provided with a notch 44, 44' and a first and second recess portion 45 in the first part 47 and a first and second recess portion 45' in the second part 46, such that each of the notches 44, 44' and opposite recess portions 45, 45' of the notched portions are arranged conform a cruciform for receiving a plunger-shaft 17, 26, when the seal 60 is in locked position.

In one embodiment, the seal 1, 50, 60 according to the invention comprises the first and second parts, wherein the notched portions 5, 5' of said first and second parts 2, 3, 47, 46 form a circular opening 35 when the seal 1, 50, 60 is in the locked position, wherein the notched portions 5, 5' are arranged conform a circular shape for receiving the plunger-shaft 25 of the plunger 14, and wherein the notched portions 5, 5' of the first and second part 2, 3, 47, 46 are each provided with a notch 30, 30' and a first and second recess portion 31, 32 in the first part 2, 47 and a first and second recess portion 33, 34 in the second part 3, 46, such that each of the notches 30, 30' and two opposite recess portions 31, 33 and two opposite recess portions 32, 34 of the notched portions are arranged conform a cruciform for receiving the plunger shaft, when the seal 1, 50, 60 is in locked position.

In one embodiment, said seal 1, 50, 60 according to the invention comprises said lock 7 provided with said slot 7', wherein an opening 9 of the slot 7' is provided with a rim portion 10, preferably a resilient rim portion 10. In one embodiment, said rim portion 10 of the opening 9 is provided with at least one notch 11, preferably two notches 11 located at opposite sides of the rim, more preferably four notches 11 located at a mutual angle of about 90°.

In one embodiment, said seal 1, 50, 60 according to the invention comprises the pin 8 provided with the bulged end portion 8' in the end portion 13, wherein the bulged end portion 8' in the end portion 13 of the pin 8 is an enlarged head encompassing the end portion 13, or is a rim, or is a harpoon of a harpoon closure encompassing the end portion 13, or is the first half of a snap closure for engagement with the second half of a snap closure located in the slot 7', or is the first half of an interlocking closure for engagement with a second half of an interlocking closure located in the slot 7'.

In one embodiment, said seal 1, 50, 60 according to the invention comprises the pin 8 provided with the bulged end portion 8' in the end portion 13, wherein the pin 8 is provided with a resilient bulged portion 8'.

In one embodiment, said seal 1, 50, 60 according to the invention comprises the pin 8, wherein the pin 8 is provided with a handle 21 arranged to release the pin 8 from the first part 2, 47 of the seal 1, 50, 60.

In one embodiment, the seal 1, 50, 60 according to the invention comprises at least one of a thermosetting polymer material selected from the group comprising polystyrene and polycarbonate, and/or one of a thermoplastic polymer material selected from acrylonitrile butadiene styrene and acrylonitrile styrene acrylate, preferably, the seal 1, 50, 60 is manufactured from polystyrene.

In one embodiment, the seal 1, 50, 60 according to the invention is manufactured from a single type of thermosetting polymer material selected from polystyrene and polycarbonate, or is manufactured from a single type of thermoplastic polymer material selected from acrylonitrile butadiene styrene and acrylonitrile styrene acrylate, preferably, the seal 1, 50, 60 is wholly manufactured from polystyrene.

Now referring to Figures 1, 2, and 11 to 13, further embodiments of the invention are illustrated. An assembly of the invention comprises a seal 1, 50, 60 according to any of the aforementioned embodiments, in particular the indicated preferred embodiments, and a syringe 15, 15'. The syringe may have a plunger shaft 17, 25, 26 having a cross sectional shape of a cylinder, or a cruciform, or a combination thereof. The seal 1, 50, 60 may have one aperture 4, 4', 20 located in the two parts 2, 3 of the seal, or preferably has at least two apertures, more preferably, two apertures for receiving the syringe grip 16, 27 of syringes, said grips having sizes and shapes within certain limits, all receivable by said apertures. Preferably, the seal of the assembly of the invention comprises a lock comprising a pin 8 provided with a handle 21 arranged to release the pin 8 from the first part 2 of the seal 1, 50, 60 by tearing or breaking a removable seal 12, *e.g.* by turning the handle 21 along the longitudinal axis of the pin 8. Furthermore, the syringe 15, 15' is preferably provided with a needle, which needle is preferably a luer-lock type of needle with regard to the attachment means for attaching the needle to the nozzle of the syringe, *i.e.* the syringe barrel. Preferably, the needle is provided with a cap, which is a releasable cap. Such cap has various functions. First, the cap closes the end portion of the needle exposed to the surrounding, thereby preventing the entrance of any substances or microbes from the surrounding into the needle and thus into the syringe barrel, and thereby preventing the liquid contained in the syringe from being released. Second, the cap prevents against possible injuries imposed by the needle.

It is one of the many benefits of the assembly 22, 23, 24 of the current invention, that a prefilled syringe can be supplied with a largely reduced risk for contamination of the liquid medicament contained by the syringe, and with a largely reduced risk for reduction of volume and quality of the liquid medicament, while at the same time the assembly of the invention provides for a ready to use prefilled syringe which is provided with a tamper proof seal further improving the safe and efficacious application of the medicament contained in the prefilled syringe.

Thus, in a particularly preferred embodiment, a syringe 15, 15' is provided with a seal 1, 50, 60 of the invention. Preferably, the syringe is a prefilled syringe. Preferably, the prefilled syringe is prefilled with a liquid medicament, such as a medicament which is relatively expensive, and/or which is difficult to manufacture, and/or which is scarce, and/or which should be administered to a subject such as a human patient at a dose and volume within relatively narrow limits, and/or which is susceptible for decay, chemical modification, *etc.,* upon exposure to air, needle material, *etc.* Typically, the liquid medicament is an aqueous pharmaceutical composition, comprising at least one active pharmaceutical ingredient. The syringe provided with a seal 1, 50, 60 of the invention, and the syringe comprised by the assembly 22, 23, 24 of the invention is typically a syringe meant for single use, such as a disposable syringe, although the single use syringe also can be a reusable syringe, according to the invention.

Since the seal 1, 50, 60 of the invention immobilizes the plunger 14 of a syringe 15, 15'relative to the syringe barrel 18, when in locked position, the syringe is conveniently provided with a (capped) needle. Currently, prefilled syringes are commonly provided with a cap, without a needle, which is a requirement since the plunger is not conveniently fixated at a predetermined position in the syringe barrel upon application of currently available seals. It is now due to the seal of the invention that prefilled syringes can be provided with a needle without the risk for unwanted and/or premature release of the contained liquid in the syringe barrel since the seal of the invention efficiently and conveniently locks the plunger firmly to the syringe barrel at a selected position. Thus, in one embodiment, an assembly 22, 23, 24 is provided comprising the seal 1, 50, 60 according to the invention and a syringe 15, 15' comprising a plunger 14 locked by said seal at a selected position such as for example a predetermined position 17' in the barrel 18 of said syringe, wherein the plunger shaft 25, 26 is essentially cylindrically shaped or the plunger shaft 17 is formed as a cruciform, or a combination thereof. Preferably, in said assembly, the syringe is a prefilled syringe filled with a liquid such as a liquid medicament, wherein the volume of the liquid preferably equals a single unit dose, and wherein preferably the prefilled syringe is provided with a luer-locked needle.

In one embodiment, a mold is provided for molding the two parts 2, 3 of the seal 1, 50, 60 of the invention, which two parts are hinged by a hinge 6, wherein the mold is for manufacturing said two hinged parts 2, 3. Preferably, the two parts 2, 3 are molded separately in two separate molds. Preferably, the parts 2, 3 are molded by applying injection molding. Preferably, the two parts 2, 3 are molded upon application of a single polymer, preferably polystyrene. Thus, in a particularly preferred embodiment, the parts 2, 3 of the seal 1, 50, 60 of the invention are molded, *e.g.* in two separate molds, wherein the parts are wholly made of polystyrene.

In one embodiment, a computer readable medium of the invention is provided, comprising computer executable instructions and 3D data representing the seal 1, 50, 60 of the invention, which computer executable instructions and 3D data representing the seal 1, 50, 60, when loaded on a computer system connected to a 3D printer, allows to 3D print the two parts 2, 3, 47, 46 of the seal 1, 50, 60. In a further embodiment, a computer system is provided comprising a processor, a memory and a 3D printer, which processor is arranged to execute instructions, stored in said memory, for 3D printing of the two parts 2, 3 of the seal 1, 50, 60 of the invention by said 3D printer.

## Claims

1. A seal (1, 50, 60) for locking a plunger (14) of a syringe (15, 15'), the seal comprising
- a first part and a second part (2, 3, 47, 46);
a hinge (6, 6a, 6b) arranged to hinge the first and second parts (2, 3, 47, 46),
each part provided with
-- at least one aperture (4, 4', 20) for receiving a grip (16) of the syringe,
-- a notched portion (5,5') for receiving the plunger (14),
the seal (1, 50, 60) further comprising
-- a lock (7) arranged at the first part and second part (2, 3, 47, 46) opposite to the hinge (6);
wherein said lock (7) is provided with
--- a pin (8) releasably connected to the first part (2, 47) through a removable seal (12), said pin (8) provided with a bulged end portion (8') in an end portion (13); and
--- a slot (7') provided in the second part (3, 46), arranged
to engage the bulged end portion (8') of the pin (8) in a locked position.

2. The seal (1, 50, 60) according to claim 1, wherein an opening (9) of the slot (7') is provided with a rim portion (10), preferably a resilient rim portion (10).

3. The seal (1, 50, 60) according to claim 2, wherein the rim portion (10) of opening (9) is provided with at least one notch (11), preferably two notches (11) located at opposite sides of the rim, more preferably four notches (11) located at an angle of about 90° relative to one another.

4. The seal (1, 50, 60) according to any one of the claims 1 to 3, wherein the bulged end portion (8') in the end portion (13) of the pin (8) is an enlarged head encompassing the end portion (13), or is a rim, or is a harpoon of a harpoon closure encompassing the end portion (13), or is the first half of a snap closure for engagement with the second half of a snap closure located in the slot (7'), or is the first half of an interlocking closure for engagement with a second half of an interlocking closure located in the slot (7').

5. The seal (1, 50, 60) according to any one of the claims 1 to 4, wherein the pin (8) is provided with a resilient bulged portion (8').

6. The seal (1, 50, 60) according to any one of the claims 1 to 5, wherein the pin (8) is provided with a handle (21) arranged to release the pin (8) from the first part (2, 47) of the seal (1, 50, 60).

7. The seal (1, 50, 60) according to any one of the claims 1 to 6, wherein said seal comprises at least one of a thermosetting polymer material selected from the group comprising polystyrene and polycarbonate, and/or one of a thermoplastic polymer material selected from acrylonitrile butadiene styrene and acrylonitrile styrene acrylate, preferably, the seal (1, 50, 60) is manufactured from polystyrene.

8. The seal (1, 50, 60) according to claim 7, wherein said seal is manufactured from a single type of thermosetting polymer material selected from polystyrene and polycarbonate, or is manufactured from a single type of thermoplastic polymer material selected from acrylonitrile butadiene styrene and acrylonitrile styrene acrylate, preferably, the seal (1, 50, 60) is wholly manufactured from polystyrene.

9. The seal (1, 50, 60) according to any one of the previous claims, wherein the notched portion (5, 5') of the first and second part (2, 3, 47, 46) is configured for stepless receiving a plunger shaft (17, 25, 26) of the plunger (14).

10. The seal (1, 50, 60) according to any one of the previous claims, wherein the first and second part (2, 3, 47, 46) each is provided with at least two apertures (4, 4', 20) for receiving the grip (16) of the syringe (15, 15').

11. An assembly (22, 23, 24) comprising the seal (1, 50, 60) according to any one of the previous claims and a syringe (15, 15').

12. The assembly according to claim 11, wherein the syringe (15, 15') is further provided with a needle connected to a nozzle of the syringe, said needle preferably provided with a releasable cap.

13. A syringe (15, 15') provided with the seal (1, 50, 60) according to any one of the claims 1 to 10.

14. A mold for molding the two parts (2, 3, 47, 46) of the seal (1, 50, 60) of any one of the claims 1 to 10.

15. Computer readable medium comprising computer executable instructions and 3D data representing the seal (1, 50, 60) of any of the claims 1 to 10, which, when loaded on a computer system connected to a 3D printer, allows to 3D print the two parts (2, 3, 47, 46) of the seal (1, 50, 60).
